# EUROPEAN PATENT APPLICATION

(11) **EP 4 461 346 A1**
(43) Date of publication of application: **13.11.2024**
(21) Application number: 22916658.2
(22) Date of filing: 26.12.2022
(51) Int. Cl.: A61N 5/10

(54) **ELECTRON PENCIL BEAM-BASED MAGNETIC ADJUSTMENT ELECTRON RAY TREATMENT APPARATUS AND SYSTEM**

(30) Priority: 03.01.2022 KR 20220000349
(71) Applicant: RADEXEL INC., Gangwon-do 24437 (KR)
(72) Inventor: JUNG, Nuri Hyun, Chuncheon-si, Gangwon-do 24363 (KR); LEE, Jeong Won, Chuncheon-si, Gangwon-do 24363 (KR); LEE, Minsik, Chuncheon-si, Gangwon-do 24229 (KR)
(74) Representative: HGF
(86) International application number: PCT/KR2022/021307
(87) International publication number: WO 2023/128517

(57) **Abstract**

The present invention relates to an electron ray treatment apparatus and system. The apparatus comprises: an electron ray output part for generating, accelerating and outputting an electron ray; a catheter part having one end connected to the electron ray output part, and receiving the electron ray, which is output from the electron ray output part, and passing the electron ray through a hollow tunnel of a catheter; a magnetic field generation part for generating a magnetic field for refracting the electron ray passing through the hollow tunnel of the catheter; and a joint driving part enabling the electron ray output part, catheter, and magnetic field generation part to move organically and freely, wherein the catheter part may adjust the movement and rotation angle of the catheter in response to the control of a control part.

## Description

### [TECHNICAL FIELD]

Embodiments of the present disclosure described herein relate to an electron ray treatment apparatus and system, and more particularly, relate to an electron ray pencil beam-based magnetic adjustment electron ray treatment apparatus and system which may irradiate electron rays to areas out of a linear irradiation range of the electron rays, such as a deep subcutaneous area and a spherical curved area of a human body of a patient while adjusting a direction and a dose of the electron rays, and thus may minimize lost electron rays and decrease power consumption.

### [BACKGROUND ART]

In recent years, with the advent of the aging era and the improvement of living standards of people, interest in early diagnosis and treatment of diseases to lead a healthy life is increasing.

In particular, a radiation treatment apparatus is a medical apparatus that uses a radiation to treat diseases and is a treatment apparatus that delays the growth of malignant tumor tissues such as cancers or destroys the malignant tumor tissues, using the radiation such as X rays, electron rays, and proton rays.

However, when a high-energy radiation is excessively irradiated to normal tissues of the human body, normal tissue cells may die, genetic defects may be caused, and cancers may be generated.

When the normal tissue and the tumor tissue are close to each other, a sufficient radiation treatment dose may not be irradiated due to side effects of the radiation.

Thus, during radiation treatment, the tumor to be destroyed should receive a sufficient radiation, and damage to the normal tissue surrounding the tumor should be minimized.

When the tumor is treated, as compared to X-ray treatment, electron ray treatment that is a type of radiation treatment may transmit a high radiation dose to a skin that is a surface of the human body and a deep portion (e.g., 1 cm to 5 cm under the skin) that is a lower portion of the skin.

Further, the radiation dose irradiated to a normal tissue that is deeper than a depth of a treatment target may be minimized.

However, in the electron ray treatment according to the related art, only a flat portion of the treatment target may be treated, and the treatment is impossible when the apparatus needs to enter a narrow passage as in an uterine cancer or when the electron rays need to be irradiated in a bent direction to a blind area out of the linear irradiation range of the electron rays as in an oral cancer.

Further, a size of the tumor or a degree of disease progression varies depending on a position of the treatment target, and thus the radiation dose needs to be adjusted. In the electron ray treatment according to the related art, it is difficult to adjust the radiation dose for each position.

In particular, in the case of an electron intraoperative radiation therapy (electron IORT) among surgery using electron rays, it is difficult to perform the electron ray treatment on a deep space through a narrow passage in a curved space formed in a spherical shape or an U shape on a surface of the treatment target as in a breast cancer, a brain tumor, or the like, minimally invasive surgery for organs such as a prostate, a lung, a liver, a pancreas, and a colon, laparoscopic/thoracic surgery, robotic surgery, or the like.

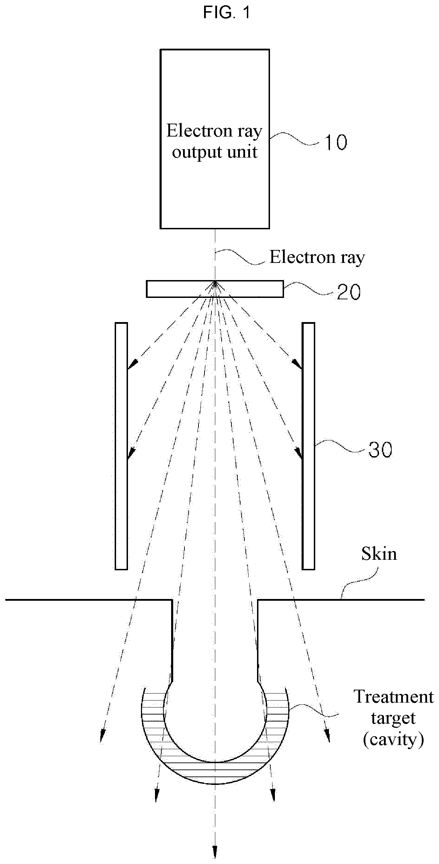

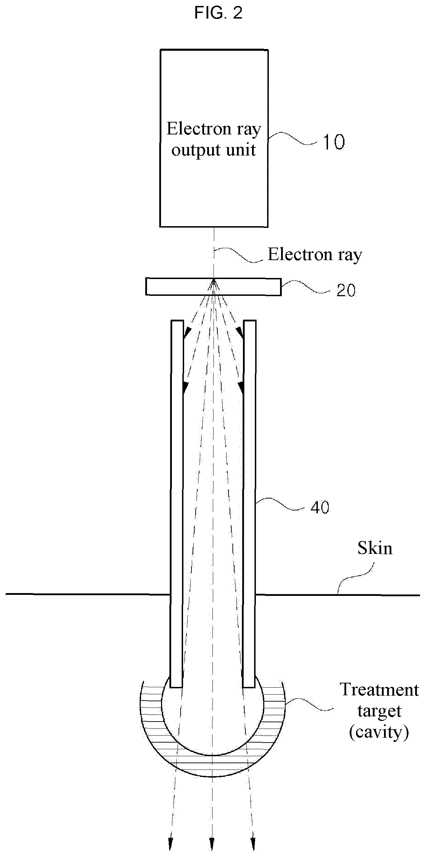

FIGS. 1 and 2 are cross-sectional views for describing an operation of an electron ray treatment apparatus according to the related art, FIG. 1 includes an electron ray output unit 10, an electron scattering unit 20, a first applicator (electron applicator/cone) 30, a skin, and a treatment target, and FIG. 2 includes the electron ray output unit, the electron scattering unit 20, a second applicator 40, the skin, and the treatment target.

First, in FIG. 1, the electron ray output unit 10 generates, accelerates, and outputs electron rays, and the electron scattering unit 20 positioned below the electron ray output unit 10 receives the electron rays, scatters electrons, and radiates a plurality of electron rays.

The first applicator 30 positioned below the electron scattering unit 20 receives and guides the plurality of electron rays scattered by the electron scattering unit 20 and shields the electron rays to prevent the electron rays from being radiated to the outside of the electron ray treatment apparatus.

The plurality of electron rays guided through the first applicator 30 are irradiated to the skin and the treatment target.

In this case, as illustrated in FIG. 1, among the plurality of irradiated electron rays, there are a large number of electron rays absorbed by the electron scattering unit 20 and the first applicator 30, and thus output loss is caused.

The electron ray output unit 10 may require a lot of power, so that the electron rays having a certain intensity or more reaches the treatment target, in consideration of the output loss.

Further, according to FIG. 1, there are many electron rays that reach the skin or a subcutaneous tissue other than the treatment target, and the normal tissues are exposed to the radiation, which is harmful to a human body of a patient.

Further, according to FIG. 2, unlike FIG. 1, a width of the second applicator 40 is too narrow, the electron rays are not guided and irradiated to the entire area of the treatment target but are guided and irradiated to only a portion of the treatment target, and thus the electron ray treatment is insufficient.

### [DETAILED DESCRIPTION OF THE INVENTION]

### [TECHINICAL PROBLEM]

Embodiments of the present disclosure provide an electron ray pencil beam-based magnetic adjustment electron ray treatment apparatus and system that may perform treatment while adjusting a direction and a dose of electron rays even when electron ray irradiation is required to a blind area out of a linear irradiation range of the electron rays when entry into a deep subcutaneous area, a spherical curved area, and a narrow passage among a treatment target of a human body of a patient is required.

The aspects of the present disclosure are not limited to the aspects described above, and those skilled in the art will clearly understand other aspects not described from the following description.

### [TECHNICAL SOLUTION]

According to an embodiment, an electron ray pencil beam-based magnetic adjustment electron ray treatment apparatus according to the present disclosure includes an electron ray output unit that generates, accelerates, and outputs an electron ray, a catheter unit of which one side is connected to the electron ray output unit and which receives the electron ray output from the electron ray output unit and allows the electron ray to pass through a hollow tunnel of a catheter, a magnetic field generator that generates a magnetic field for refracting the electron ray passing through the hollow tunnel of the catheter, and a joint driving part that provides a degree of freedom of organic movement of the electron ray output unit, the catheter, and the magnetic field generator, wherein the catheter unit adjusts movement and a rotational angle of the catheter in response to control of a controller.

In this case, the catheter unit may adjust distribution of the electron ray irradiated to a treatment target by adjusting a refraction angle of the magnetic field generator in a state in which a position of the catheter is fixed at a certain depth in response to the control of the controller.

Further, the catheter unit may adjust distribution of the electron ray irradiated to a treatment target while moving the magnetic field generator together according to vertical movement, left-right movement, and rotation of the catheter in response to the control of the controller.

Meanwhile, the electron ray output unit may output a pencil beam-shaped electron ray.

Further, the magnetic field generator may be equipped with a first magnetic pole and a second magnetic pole in the form of one of a permanent magnet, an electromagnet, or a hybrid magnet in which the permanent magnet and the electromagnet are used together, at both ends of one side of the catheter. That is, when the magnetic field generator is in form of the permanent magnet, the magnetic field generator may adjust a refraction angle of the electron ray by adjusting positions of the first magnetic pole and the second magnetic pole and a distance between the first magnetic pole and the second magnetic pole. Further, when the magnetic field generator is in the form of the electromagnet, the magnetic field generator may be a pulsed electromagnet, may generate a magnetic field in synchronization with an electron ray pulse, and may adjust a refraction angle of the electron ray by adjusting an intensity and direction of the generated magnetic field.

According to an embodiment, an electron ray pencil beam-based magnetic adjustment electron ray treatment system includes an electron ray treatment apparatus that generates and accelerates an electron ray, outputs the electron ray in the form of a pencil beam, and allows the output electron ray to pass through a hollow tunnel of a catheter and to be irradiated to a treatment target, a controller that is spaced apart from the electron ray treatment apparatus and remotely controls the electron ray treatment apparatus, and a power supply unit that supplies a power voltage required for the electron ray treatment apparatus in response to the control of the controller, wherein the electron ray treatment apparatus adjusts movement and a rotational angle of the catheter in response to the control of the controller and drives the catheter.

In this case, the controller may control an energy intensity, a dose, a speed, and an output timing of the electron ray and a magnitude, a direction, and an output timing of a magnetic field generated by a magnetic field generator inside the electron ray treatment apparatus.

Further, the controller may perform a control to adjust distribution of the electron ray irradiated to the treatment target by adjusting a refraction angle of the magnetic field generator in a state in which a position of the catheter is fixed at a certain depth.

Further, the controller may perform a control to adjust distribution of the electron ray irradiated to the treatment target while moving the magnetic field generator together according to vertical movement, left-right movement, and rotation of the catheter.

Further, the magnetic field generator may be equipped with a first magnetic pole and a second magnetic pole in the form of one of a permanent magnet, an electromagnet, or a hybrid magnet in which the permanent magnet and the electromagnet are used together, at both ends of one side of the catheter. That is, when the magnetic field generator is in form of the permanent magnet, the magnetic field generator may adjust a refraction angle of the electron ray by adjusting positions of the first magnetic pole and the second magnetic pole and a distance between the first magnetic pole and the second magnetic pole. Further, when the magnetic field generator is in the form of the electromagnet, the magnetic field generator may be a pulsed electromagnet, may generate a magnetic field in synchronization with an electron ray pulse, and may adjust a refraction angle of the electron ray by adjusting an intensity and direction of the generated magnetic field.

Further, the electron ray treatment apparatus may be manufactured as a modular apparatus that receives the electron ray through an electron ray output unit while spaced apart from the electron ray output unit that outputs the electron ray.

### [ADVANTAGEOUS EFFECTS OF THE INVENTION]

According to the present disclosure, an electron scattering unit and an applicator are unnecessary and loss or leakage of electron rays may be minimized. Thus, when the same electron ray output is targeted, power consumed in an electron ray output unit may be reduced, and when the same power is consumed, output of the electron rays may be increased.

Further, electron ray treatment may be sufficiently performed not only on a flat portion of a human body of a patient but also on a subcutaneous area and a spherical curved area, and the electron ray treatment may be accurately and appropriately performed while a radiation dose is adjusted on an area of a treatment target area, which is intended by a medical staff.

Further, because pencil beam-shaped electron rays are generated, a size of a space that requires a magnetic field may be decreased, the magnetic field generator may be miniaturized, and thus, a treatment apparatus may enter a narrow space inside the human body of the patient.

Further, when the treatment apparatus is used during radiation treatment during surgery, the risk of radiation exposure to the patient and the medical staff is reduced due to a decrease in leakage electron rays, and a radiation treatment time during the surgery is shortened due to an increase in the electron ray output.

The effects of the present disclosure are not limited to the effects described above, and those skilled in the art will clearly understand other effects not described from the following description.

### [DESCRIPTION OF THE DRAWINGS]

FIGS. 1 and 2 are cross-sectional view for describing an operation of an electron ray treatment apparatus according to the related art.
FIG. 3 is a schematic diagram of an electron ray pencil beam-based magnetic adjustment electron ray treatment system according to the present disclosure.
FIG. 4 is a schematic cross-sectional view for describing an operation of an electron ray pencil beam-based magnetic adjustment electron ray treatment apparatus according to the present disclosure.
FIG. 5 is a view illustrating an experimental model produced for a magnetic adjustment electron ray treatment apparatus (100) and a cured treatment target illustrated in FIG. 4.
FIG. 6 is cross-sectional views of electron rays of various aspects, which are irradiated to a flat treatment target (a) and a curved treatment target (b) according to vertical movement of a magnetic field generator (140) in the magnetic adjustment electron ray treatment apparatus illustrated in FIG. 4.
FIG. 7 is a cross-sectional view of electron rays of various aspects, which are irradiated to a treatment target according to left-right movement of the magnetic field generator (140) in the magnetic adjustment electron ray treatment apparatus illustrated in FIG. 4.
FIG. 8 is a cross-sectional views of electron rays of various aspects, which are irradiated to the flat treatment target (a) and the curved treatment target (b) according to a refraction angle of the magnetic field generator (140) in the magnetic adjustment electron ray treatment apparatus illustrated in FIG. 4.
FIGS. 9A to 9C are views illustrating a state in which the magnetic adjustment electron ray treatment apparatus illustrated in FIG. 4 is manufactured in a modular form and is equipped with the magnetic field generator when viewed from various angles.
FIG. 10 is a view illustrating that electron rays due to a magnetic field may be refracted in various angles using an electron ray output unit (110), a catheter (130), and the magnetic field generator (140) illustrated in FIG. 4.
FIGS. 11A to 11C are views illustrating electron ray traces displayed on a subject (400) as a result of the experiment illustrated in FIG. 8.

### [BEST MODE]

Advantages and features of the present disclosure and a method of achieving the advantages and the features will become apparent with reference to embodiments described below in detail together with the accompanying drawings. However, the inventive concept is not limited to the embodiments described below but may be implemented in various forms, and the present embodiments merely make the disclosure of the inventive concept complete and are provided to completely inform the scope of the inventive concept to those skilled in the art to which the inventive concept belongs, and the inventive concept is merely defined by the scope of the appended claims.

Terms used in the present specification are intended to describe an embodiment and are not intended to limit the inventive concept. In the specification, a singular form also includes a plural form unless specifically mentioned in a phrase. The terms "comprises" and/or "comprising" used in the specification do not exclude the presence or addition of one or more other components other than the mentioned components. Throughout the specification, the same reference numerals refer to the same components, and the term "and/or" includes each and all combinations of one or more of components mentioned. Although "first," "second," and the like are used to describe various components, it is apparent that these components are not limited by these terms. These terms are only used to distinguish one component from another component. Thus, it is apparent that a first component mentioned below may be a second component within the technical spirit of the inventive concept.

Unless otherwise defined, all the terms (including technical and scientific terms) used herein may be used as meanings that may be commonly understood by those skilled in the art to which the inventive concept belongs. Further, terms defined in a commonly used dictionary are not interpreted ideally or excessively unless explicitly and specifically defined.

Spatially relative terms such as "below", "beneath", "lower", "above", and "upper" may be used to easily describe a correlation between a first component and a second component as illustrated in the drawings. The spatially relative terms should be understood as terms including different directions of components during use or operation in addition to directions illustrated in the drawings. For example, when the first component illustrated in the drawings is turned over, the first component described to be located "below" or "beneath" the second component may be placed "above" the second component. Thus, the exemplary term "below" may include both downward and upward directions. The components may be oriented in other directions, and accordingly, the spatially relative terms may be interpreted according to the orientation.

Hereinafter, embodiments of the inventive concept will be described in detail with reference to the accompanying drawings.

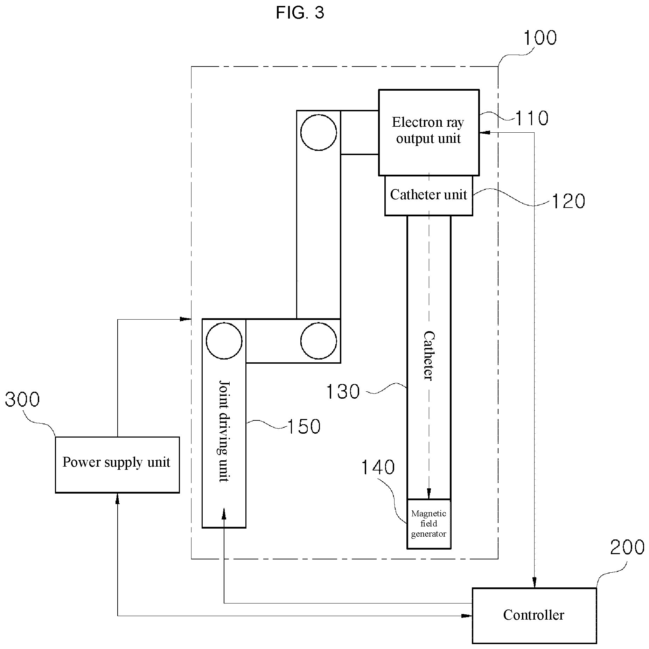

FIG. 3 is a schematic diagram of an electron ray pencil beam-based magnetic adjustment electron ray treatment system which includes an electron ray treatment apparatus 100, a controller 200, and a power supply unit 300.

The electron ray treatment apparatus 100 includes an electron ray output unit 110, a catheter unit 120, a catheter 130, a magnetic field generator 140, and a joint driving part 150.

The electron ray pencil beam-based magnetic adjustment electron ray treatment system according to the present disclosure will be briefly described below with reference to FIG. 3.

The electron ray treatment apparatus 100 generates and accelerates pencil beam-shaped electron rays, allows the pencil beam-shaped electron rays to pass through a hollow tunnel of the catheter 130, and irradiates the pencil beam-shaped electron rays to a treatment target.

The controller 200 is a user terminal that is spaced apart from the electron ray treatment apparatus 100 and may remotely control the electron ray treatment apparatus 100 and the power supply unit 300, and includes input means such as a keyboard and a mouse and output means such as a display unit.

That is, the controller 200 controls an energy intensity, a dose, a speed, and an output timing of electron rays output from the electron ray output unit 110 and an intensity, a direction, and an output timing of a magnetic field generated by the magnetic field generator 140 in the electron ray treatment apparatus 100.

The power supply unit 300 supplies a power voltage required for the electron ray treatment apparatus 100 in response to the control of the controller 200.

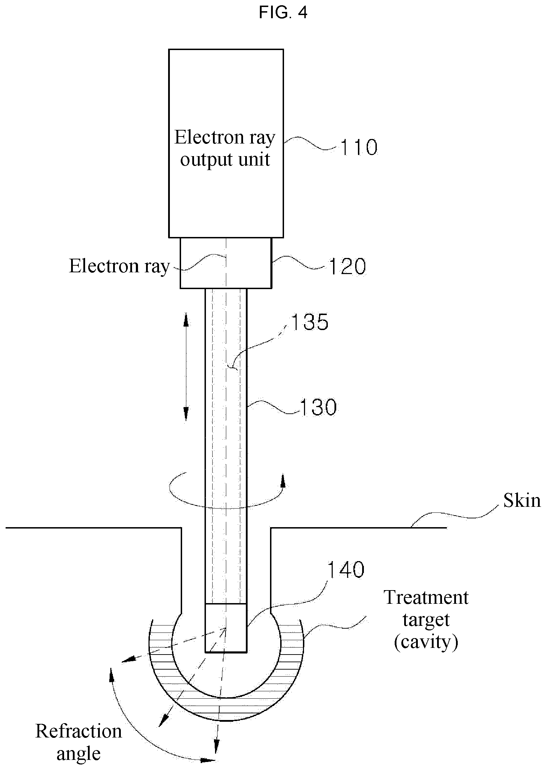

FIG. 4 is a schematic cross-sectional view for describing an operation of the electron ray pencil beam-based magnetic adjustment electron ray treatment apparatus according to the present disclosure, which includes the electron ray output unit 110, the catheter unit 120, the catheter 130, the magnetic field generator 140, a skin, and a treatment target.

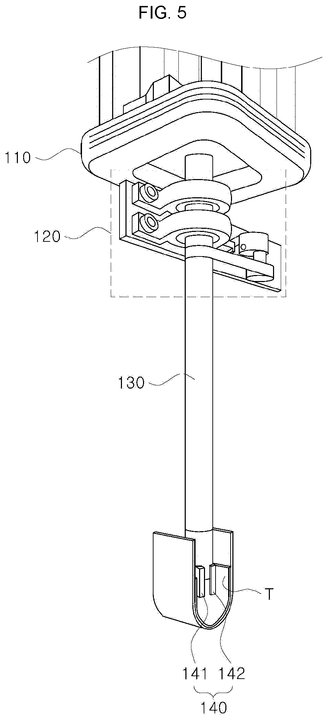

FIG. 5 is a picture of an experimental model produced for the electron ray treatment apparatus 100 and a curved treatment target "T" illustrated in FIG. 4, which includes the electron ray output unit 110, the catheter unit 120, the catheter 130, the magnetic field generator 140, and a treatment target model.

In the treatment target model, a U-shaped white area positioned on a lower side is obtained by modeling an internal space of a human body of a patient, and a green area "T" inside the white area is obtained by modeling the treatment target that requires radiation treatment.

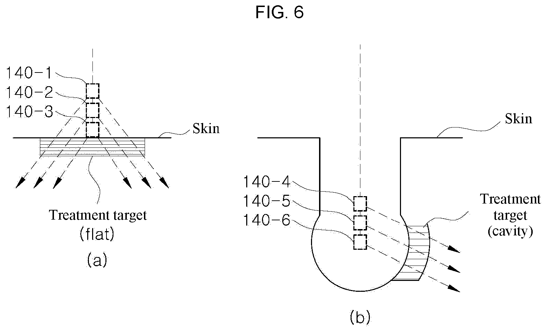

FIG. 6 is a cross-sectional view of electron rays of various aspects, which are irradiated to a flat treatment target "a" and a curved treatment target "b" according to vertical movement of the magnetic field generator 140 in the magnetic adjustment electron ray treatment apparatus 100 illustrated in FIG. 4, which includes magnetic field generators 140-1 to 140-6, the skin, and the treatment target.

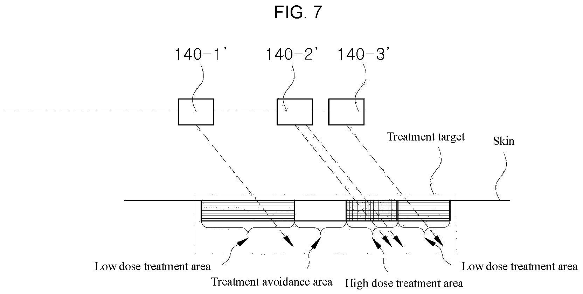

FIG. 7 is a cross-sectional view of electron rays of various aspects, which are irradiated to the treatment target according to left-right movement of the magnetic field generator 140 in the magnetic adjustment electron ray treatment apparatus 100 illustrated in FIG. 4, which includes magnetic field generators 140-1' to 140-3', the skin, and the treatment target.

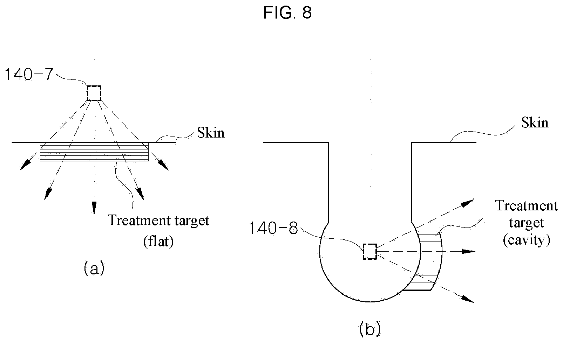

FIG. 8 is a cross-sectional view of electron rays of various aspects, which are irradiated to the flat treatment target "a" and the curved treatment target "b" according to a refraction angle of the magnetic field generator 140 in the magnetic adjustment electron ray treatment apparatus 100 illustrated in FIG. 4, which includes magnetic field generators 140-7 and 140-8, the skin, and the treatment target.

Configurations and functions of components of the electron ray pencil beam-based magnetic adjustment electron ray treatment apparatus according to the present disclosure will be briefly described below with reference to FIGS. 3 to 8.

The electron ray output unit 110 generates, accelerates, and outputs electron rays.

One side (upper side) of the catheter unit 120 is connected to a lower portion of the electron ray output unit 110, the other side (lower side) of the catheter unit 120 is connected to the catheter 130, and the catheter unit 120 receives the electron rays output from the electron ray output unit 110 and allows the electron rays to pass through a hollow tunnel 135 of the catheter 130.

The magnetic field generator 140 is connected to one side (lower side) of the catheter 130 and generates a magnetic field for refracting the electron rays passing through the hollow tunnel 135 of the catheter 130.

One side of the joint driving part 150 is connected to the electron ray output unit 110, and thus the joint driving part 150 provides the degree of freedom of organic movement of the electron ray output unit 110, the catheter 130, and the magnetic field generator 140.

In this case, the catheter unit 120 drives the catheter 130 by adjusting movement and a rotational angle of the catheter 130 in response to the control of the controller 200.

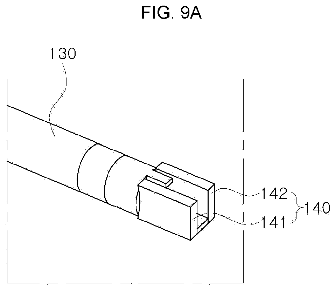

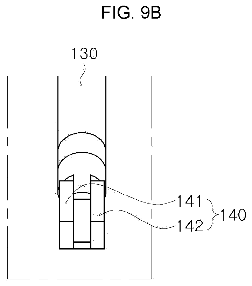

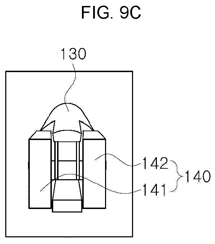

FIGS. 9A to 9C are views illustrating a state in which the magnetic adjustment electron ray treatment apparatus illustrated in FIG. 4 is manufactured in a modular form and is equipped with the magnetic field generator when viewed from various angles, FIG. 9A illustrates a state in which a side portion of the magnetic field generator in the magnetic adjustment electron ray treatment apparatus illustrated in FIG. 4 is photographed toward below at a predetermined (or preset) angle, FIG. 9B illustrates a state in which an upper portion of the magnetic generator is photographed from below at a predetermined (preset) angle, FIG. 9C illustrates a state in which a lower portion of the magnetic adjustment electron ray treatment apparatus is photographed from a front side, which include the catheter 130 and the magnetic field generator 140, and the magnetic field generator 140 includes a first magnetic pole 141 and a second magnetic pole 142.

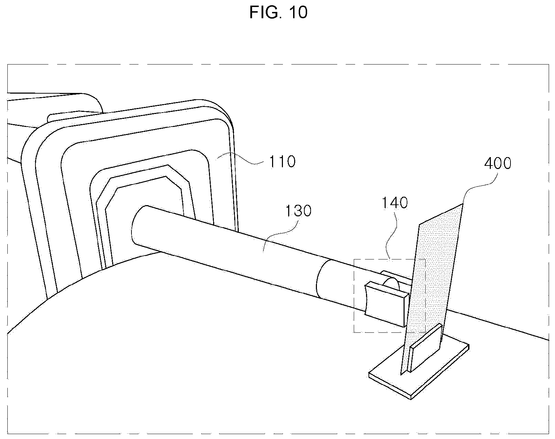

FIG. 10 illustrates that the electron rays may be refracted at various angles by a magnetic field using the electron ray output unit 110, the catheter 130, and the magnetic field generator 140 illustrated in FIG. 4, which includes the electron ray output unit 110, the catheter 130, the magnetic field generator 140, and a subject 400.

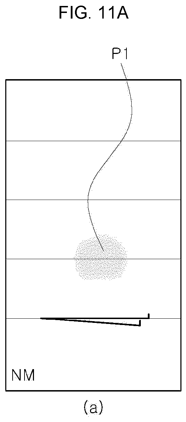

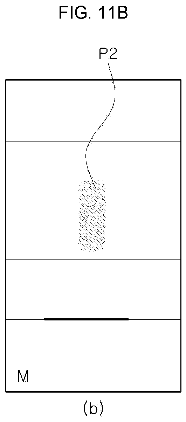

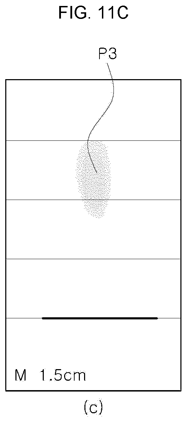

FIG. 11 is a picture of electron ray traces displayed on the subject 400 as a result of the experiment illustrated in FIG. 10.

The electron ray pencil beam-based magnetic adjustment electron ray treatment apparatus according to the present disclosure will be described below in detail with reference to FIGS. 3 to 11.

In FIG. 3, the electron ray output unit 110 generates pencil beam-shaped electron rays and accelerates and outputs the generated pencil beam-shaped electron rays.

In this case, energy of the electron rays may be set to 1 MeV to 15 MeV, and a diameter thereof may be set to 0.1 cm to 3 cm.

The catheter unit 120 is connected to a lower portion of the electron ray output unit 110, receives the electron rays output from the electron ray output unit 110 to adjust the movement and the rotational angle of the catheter 130 and adjust the refraction angle of the magnetic field generator 140.

The catheter 130 has the hollow tunnel 135, which is a cylindrical empty space, in a center thereof, is connected to a lower portion of the catheter unit 120, and transmits the electron rays while rotating in response to control of the catheter unit 120 as illustrated in FIG. 4.

In this case, the catheter 130 may be driven independently of the electron ray output unit 110 in a module form and move individually or may be integrally fixed to the electron ray output unit 110 and vertically move together.

Further, a gas composition may be changed to helium gas or vacuum to decrease scattering of the electron rays passing through an interior of the catheter 130.

The magnetic field generator 140 is connected to one end of the catheter 130 and generates a magnetic field for refracting the electron rays passing through an internal space of the catheter 130 while adjusting the refraction angle in response to the control of the catheter unit 120.

As illustrated in FIG. 4, the electron rays of which the refraction angles are adjusted by the magnetic field generated by the magnetic field generator 140 are evenly irradiated to various portions of the treatment target to control distribution of the electron ray irradiation.

In this case, the magnetic field generator 140 may also adjust the distribution of the electron ray irradiation while moving together in vertical movement of the catheter 130 and may also adjust the distribution of the electron ray irradiation while the refraction angle is adjusted in a state in which the position of the catheter 130 is fixed at a certain depth.

That is, as illustrated in FIG. 6A, when the treatment target is present on a surface of the human body of the patient (e.g., in skin cancer, surgery of a large organ, or the like), the electron rays may be irradiated as the position of the magnetic field generator 140 is changed to a first depth to a third depth 140-1 to 140-3 according to the vertical movement of the catheter 130, and thus all portions of the treatment target having a flat shape may be evenly treated.

Further, as illustrated in FIG. 6B, when the treatment target is present on a spherical curved surface distant from the skin of the patient by a certain depth (e.g., in breast cancer, brain tumor, or the like), the electron rays may be irradiated as the position of the magnetic field generator 140 is changed to a fourth depth to a sixth depth 140-4 to 140-6 according to the vertical movement of the catheter 130, and thus all portions of the treatment target may be evenly treated.

Alternatively, as illustrated in FIG. 7, as the position of the magnetic field generator 140 is changed from a first position to a third position (140-1' to 140-3') according to the left-right movement of the catheter 130, a radiation dose generated by the magnetic field generator 140 is adjusted to a low dose that is smaller than a at least one preset standard or a high dose that is greater than the standard or the electron rays are irradiated while avoiding a specific area, and thus the treatment target having a flat shape (or a curved shape) may be treated at different intensities for each position.

Alternatively, as illustrated in FIG. 8A, even when the treatment target is present on a skin surface of the patient, the electron rays may be irradiated while the refraction angle of the magnetic field generator 140 is variously adjusted in a state in which the position of the catheter 130 is fixed at a preset depth 140-7, and thus all portions of the treatment target having a flat shape may be evenly treated.

Further, as illustrated in FIG. 6B, even when the treatment target is present on a spherical curved surface distant from the skin of the patient by a certain depth, the electron rays may be irradiated while the refraction angle of the magnetic field generator 140 is variously adjusted in a state in which the position of the catheter 130 is fixed at a preset depth 140-8, and thus all portions of the treatment target having a spherical curved shape may be evenly treated.

Accordingly, electron ray treatment may be sufficiently performed even in a deep subcutaneous area and a spherical curved area of the human body of the patient, and the electron ray treatment may be accurately and appropriately performed while the radiation dose is entirely or partially adjusted in an area of a treatment target area, which is intended by a medical staff.

In this case, to secure a sufficient treatment space, an auxiliary medical device such as a balloon or a plastic polyhedron may be attached to a space close to the treatment target.

Further, generally, the medical staff should be in a separate space distant from the patient during the radiation treatment during surgery due to the risk of radiation exposure to the medical staff, and thus the risk of the patient is increased as a radiation treatment time during the surgery is increased. However, according to the present disclosure, the radiation treatment time during the surgery may be shortened, thereby reducing the risk of the patient.

The magnetic field generator 140 may be configured in one form of a permanent magnet, an electromagnet, or a hybrid magnet in which the permanent magnet and the electromagnet are used together.

In this case, when the magnetic field generator 140 is configured in the form of the permanent magnet, refraction angles of the electron rays generated by the electron ray output unit 110 may be adjusted by adjusting speeds of the electron rays.

Further, when the magnetic field generator 140 is configured in the form of the permanent magnet, positions of the first magnetic pole 141 and the second magnetic pole 142 may be adjusted, the first magnetic pole 141 and the second magnetic pole 142 may deviate from a passage through which the electron rays pass, and thus an influence of the magnetic field from the magnetic field generator 140 on the electron rays may be decreased.

Further, when the magnetic field generator 140 is configured in the form of the permanent magnet, a distance between the first magnetic pole 141 and the second magnetic pole 142 may be adjusted to adjust the refraction angles of the electron rays. That is, the distance between the first magnetic pole 141 and the second magnetic pole 142 is adjusted, an intensity of the magnetic field is decreased when the distance is increased, and the intensity of the magnetic field is increased when the distance is decreased.

Meanwhile, when the magnetic field generator 140 is configured in the electromagnet, the magnetic field generator 140 is a pulsed electromagnet and generates a magnetic field in synchronization with an electron ray pulse, and the refraction angles of the electron rays may be adjusted by adjusting an intensity and direction of the generated magnetic field.

Therefore, heat generation may be decreased when the same magnetic field is generated, and magnetic field output may be increased under the same heat generation condition.

The intensity of the magnetic field generated by the magnetic field generator 140 may be set to 0.1 Tesla to 1.0 Tesla.

The controller 200 not only controls the operations of the electron ray output unit 110 and the magnetic field generator 140 but also adjusts a moving distance and a speed and the rotational angle and an angular speed of the catheter 130 and the refraction angle of the magnetic field generator 140 and adjusts the radiation dose for each position of an affected area so that a preset radiation dose is transmitted to each portion of the treatment target. Therefore, the electron ray treatment may be performed for portions having various shapes and positions such as spherical shapes, narrow passages, and bent portions, which are impossible to treat with an electron ray treatment apparatus according to the related art.

One side of the joint driving part 150 is connected to the electron ray output unit 110, the other side thereof is connected to the power supply unit 300, and thus the joint driving part 150 provides the degree of freedom so that the electron ray output unit 110, the catheter 130, and the magnetic field generator 140 move organically.

In this way, in the magnetic adjustment electron ray treatment apparatus according to the present disclosure, an electron scattering unit and an applicator provided in the electron ray treatment apparatus according to the related art are unnecessary. Thus, among the electron rays generated by the electron ray output unit 110, there are no electron rays absorbed by the electron scattering unit and the applicator, so that loss of the electron rays may be minimized. Thus, when the same output is targeted, power consumed in the electron ray output unit 110 may be reduced, and when the same power is consumed, the output of the electron rays may be increased.

Further, the electron ray treatment apparatus 100 according to the present disclosure is based on an electron ray pencil beam. As compared to the electron ray treatment according to the related art in which a large overall area (e.g., 40x40 cm² or more) is generally and simultaneously treated, the pencil beam-based electron ray treatment according to the present disclosure is characterized by intensively irradiating a narrow unit area (for example, 0.5x0.5 cm² or less) and then sequentially irradiating a subsequent position to treat the entire area.

Further, considering that a radiation dose absorption rate in the electron scattering unit according to the related art is about 50%, and at the same time, a treated area is reduced by 6,400 times (multiple of the pencil beam treatment area of 0.5×0.5 cm² compared to the treatment area according to the related art of 40x40 cm²), it may be identified that a treatment speed based on each unit area of 0.5×0.5 cm² is increased by about 10,000 times compared to the related art.

Thus, considering that output is 0.1 Gy/s in general radiation treatment and is 10 Gy/s under a special condition, the electron ray treatment apparatus 100 according to the present disclosure based on the electron ray pencil beam may output 1,000 Gy/s to 100,000 Gy/s, and thus a flash effect known to occur during treatment at a speed of 40 Gy/s or higher may be obtained. Further, when the output is partially decreased, the electron ray treatment apparatus 100 may be manufactured in a small size, may move, and thus may be used for the radiation treatment during the surgery.

The electron ray pencil beam may enter a narrow space through the catheter 130. As the electron ray pencil beam is irradiated with a small diameter, a size of a space requiring the magnetic field is also decreased, and thus the magnetic field generator 140 may be miniaturized. Both the catheter 130 and the magnetic field generator 140 may enter a narrow space inside the human body of the patient.

In addition, the electron ray treatment apparatus 100 according to the present disclosure may be manufactured as a stand-alone apparatus including the electron ray output unit 110 as illustrated in FIGS. 9A to 9C as well as may be manufactured as a modular apparatus that may be spaced apart from electron ray generating equipment according to the related art and receive the electron rays as illustrated in FIG. 5.

Further, as illustrated in FIGS. 9A to 9C, the first magnetic pole 141 and the second magnetic pole 142 of the magnetic field generator 140 are respectively mounted on both ends of one side of the catheter 130 having a certain shape.

As illustrated in FIG. 10, the electron ray treatment apparatus 100 according to the present disclosure may generate the electron rays while integrally formed with the electron ray output unit 110, and the controller 200 may irradiate the electron rays while variously adjusting the refraction angle of the magnetic field generator 140.

As illustrated in FIG. 10, when the electron ray treatment apparatus 100 is integrally formed with the electron ray output unit 110, rotation of the catheter 130 and the magnetic field generator 140 is controlled by the catheter unit 120, and in the vertical movement, the entire electron ray treatment apparatus 100 moves together.

As illustrated in FIG. 11A, when no magnetic field is generated, an electron ray trace displayed on a subject is displayed at a first position P1 that is the lowest position.

As illustrated in FIG. 11B, when the magnetic field is generated by the magnetic field generator 140 in response to the control of the controller 200, the electron ray trace is displayed at a second position P2 that is a middle height.

As illustrated in FIG. 11C, when the electron ray is irradiated in a state in which a height at which the electron ray is irradiated is moved upward by 1.5 cm by adjusting the refraction angle of the magnetic field generator 140 and/or adjusting the position of the magnetic field generator 140 under the control of the controller 200, it may be identified that the electron ray trace is displayed at a third position P3 that is the highest position.

In this way, the present disclosure provides an electron ray pencil beam-based electron ray treatment apparatus and system that may perform the treatment while adjusting a direction and a dose of the electron rays even when the electron ray irradiation is required to a blind area out of a linear irradiation range of the electron rays when entry into the deep subcutaneous area, the spherical curved area, and a narrow passage among the treatment target of the human body of the patient is required.

Therefore, the electron scattering unit and the applicator are unnecessary, and loss or leakage of the electron rays may be minimized. Thus, when the same electron ray output is targeted, power consumed in the electron ray output unit may be reduced, and when the same power is consumed, the output of the electron rays may be increased.

Further, the electron ray treatment may be sufficiently performed not only on a flat portion of the human body of the patient but also on the deep subcutaneous area and the spherical curved area, and the electron ray treatment may be accurately and appropriately performed while the radiation dose is adjusted on an area of the treatment target area, which is intended by the medical staff.

Further, because the pencil beam-shaped electron rays are generated, the size of the space that requires the magnetic field may be decreased, the magnetic field generator may be miniaturized, and thus, the treatment apparatus may enter the narrow space inside the human body of the patient.

Further, when the treatment apparatus is used during the radiation treatment during the surgery, the risk of radiation exposure to the patient and the medical staff is reduced due to a decrease in leakage electron rays, and the radiation treatment time during the surgery is shortened due to an increase in the electron ray output.

Hereinabove, the embodiments of the present disclosure have been described with reference to the accompanying drawings. However, those skilled in the art to which the present disclosure pertains can understand that the present disclosure can be implemented in other specific forms without changing the technical spirit or essential features thereof. Therefore, it should be understood that the embodiments described above are illustrative but not limiting in all aspects.

## Claims

1. An electron ray pencil beam-based magnetic adjustment electron ray treatment apparatus comprising:
an electron ray output unit configured to generate, accelerate, and output an electron ray;
a catheter unit of which one side is connected to the electron ray output unit and which receives the electron ray output from the electron ray output unit and allows the electron ray to pass through a hollow tunnel of a catheter;
a magnetic field generator configured to generate a magnetic field for refracting the electron ray passing through the hollow tunnel of the catheter; and
a joint driving part configured to provide a degree of freedom of organic movement of the electron ray output unit, the catheter, and the magnetic field generator,
wherein the catheter unit adjusts movement and a rotational angle of the catheter in response to control of a controller.

2. The electron ray pencil beam-based magnetic adjustment electron ray treatment apparatus of claim 1, wherein the catheter unit adjusts distribution of the electron ray irradiated to a treatment target by adjusting a refraction angle of the magnetic field generator in a state in which a position of the catheter is fixed at a certain depth in response to the control of the controller.

3. The electron ray pencil beam-based magnetic adjustment electron ray treatment apparatus of claim 1, wherein the catheter unit adjusts distribution of the electron ray irradiated to a treatment target while moving the magnetic field generator together according to vertical movement, left-right movement, and rotation of the catheter in response to the control of the controller.

4. The electron ray pencil beam-based magnetic adjustment electron ray treatment apparatus of claim 1, wherein the electron ray output unit outputs a pencil beam-shaped electron ray.

5. The electron ray pencil beam-based magnetic adjustment electron ray treatment apparatus of claim 2, wherein the magnetic field generator is equipped with a first magnetic pole and a second magnetic pole in the form of one of a permanent magnet, an electromagnet, or a hybrid magnet in which the permanent magnet and the electromagnet are used together, at both ends of one side of the catheter.

6. The electron ray pencil beam-based magnetic adjustment electron ray treatment apparatus of claim 5, wherein, when the magnetic field generator is in form of the permanent magnet, the magnetic field generator adjusts a refraction angle of the electron ray by adjusting positions of the first magnetic pole and the second magnetic pole and a distance between the first magnetic pole and the second magnetic pole.

7. The electron ray pencil beam-based magnetic adjustment electron ray treatment apparatus of claim 5, wherein, when the magnetic field generator is in the form of the electromagnet, the magnetic field generator is a pulsed electromagnet, generates a magnetic field in synchronization with an electron ray pulse, and adjusts a refraction angle of the electron ray by adjusting an intensity and direction of the generated magnetic field.

8. An electron ray pencil beam-based magnetic adjustment electron ray treatment system comprising:
an electron ray treatment apparatus configured to generate and accelerate an electron ray, output the electron ray in the form of a pencil beam, and allow the output electron ray to pass through a hollow tunnel of a catheter and to be irradiated to a treatment target;
a controller spaced apart from the electron ray treatment apparatus and configured to remotely control the electron ray treatment apparatus; and
a power supply unit configured to supply a power voltage required for the electron ray treatment apparatus in response to control of the controller,
wherein the electron ray treatment apparatus adjusts movement and a rotational angle of the catheter in response to the control of the controller.

9. The electron ray pencil beam-based magnetic adjustment electron ray treatment system of claim 8, wherein the controller controls an energy intensity, a dose, a speed, and an output timing of the electron ray and a magnitude, a direction, and an output timing of a magnetic field generated by a magnetic field generator inside the electron ray treatment apparatus.

10. The electron ray pencil beam-based magnetic adjustment electron ray treatment system of claim 9, wherein the controller performs a control to adjust distribution of the electron ray irradiated to the treatment target by adjusting a refraction angle of the magnetic field generator in a state in which a position of the catheter is fixed at a certain depth.

11. The electron ray pencil beam-based magnetic adjustment electron ray treatment system of claim 9, wherein the controller performs a control to adjust distribution of the electron ray irradiated to the treatment target while moving the magnetic field generator together according to vertical movement, left-right movement, and rotation of the catheter.

12. The electron ray pencil beam-based magnetic adjustment electron ray treatment system of claim 9, wherein the magnetic field generator is equipped with a first magnetic pole and a second magnetic pole in the form of one of a permanent magnet, an electromagnet, or a hybrid magnet in which the permanent magnet and the electromagnet are used together, at both ends of one side of the catheter.

13. The electron ray pencil beam-based magnetic adjustment electron ray treatment system of claim 12, wherein, when the magnetic field generator is in form of the permanent magnet, the magnetic field generator adjusts a refraction angle of the electron ray by adjusting positions of the first magnetic pole and the second magnetic pole and a distance between the first magnetic pole and the second magnetic pole.

14. The electron ray pencil beam-based magnetic adjustment electron ray treatment system of claim 12, wherein, when the magnetic field generator is in the form of the electromagnet, the magnetic field generator is a pulsed electromagnet, generates a magnetic field in synchronization with an electron ray pulse, and adjusts a refraction angle of the electron ray by adjusting an intensity and direction of the generated magnetic field.

15. The electron ray pencil beam-based magnetic adjustment electron ray treatment system of claim 8, wherein the electron ray treatment apparatus is manufactured as a modular apparatus configured to receive the electron ray through an electron ray output unit while spaced apart from the electron ray output unit configured to output the electron ray.
